# EUROPÄISCHE PATENTSCHRIFT

(11) **EP 0 638 547 B1**
(45) Veröffentlichungstag und Bekanntmachung des Hinweises auf die Patenterteilung: **16.08.2000**
(21) Anmeldenummer: 94810449.2
(22) Anmeldetag: 29.07.1994
(51) Int. Cl.: C07C 271/12, C07C 271/24, C07C 271/28, C07C 271/34, C07C 271/36, C07C 271/38, C07D 303/16, C08J 3/28, G03F 7/031

(54) **Neue urethangruppenhaltige (Meth)Acrylate**
(Meth)acrylates containing urethanes
(Méth)acrylates contenant d'uréthanes

(30) Priorität: 09.08.1993 CH 237093
(43) Veröffentlichungstag der Anmeldung: 15.02.1995
(73) Patentinhaber: Ciba Specialty Chemicals Holding Inc., 4057 Basel (CH)
(72) Erfinder: Steinmann, Bettina, Dr., CH-1724 Praroman (CH); Wolf, Jean-Pierre, Dr., 1791 Courtaman (CH); Schulthess, Adrian, Dr., 1734 Tentlingen (CH); Hunziker, Max, Dr., 3186 Düdingen (CH)

(56) Entgegenhaltungen:
- EP-A- 0 111 259
- EP-A- 0 264 551
- CHEMICAL ABSTRACTS, vol. 96, no. 26, 1982, Columbus, Ohio, US; abstract no. 218764f, & JP-A-56 169 065 (MATSUSHITA ELECTRIC WORKS, LTD) 25. Dezember 1981
- CHEMICAL ABSTRACTS, vol. 84, no. 24, 1976, Columbus, Ohio, US; abstract no. 165551c, & JP-A-49 050 090 (LOCTITE (IRELAND) LTD.) 15. Mai 1974

## Beschreibung

Die vorliegende Erfindung betrifft neue urethangruppenhaltige (Meth)Acrylate, deren Herstellung und Verfahren zum Polymerisieren dieser Verbindungen mittels aktinischer Bestrahlung und deren Einsatz z.B. in der Stereolithographie zur Herstellung von dreidimensionalen Gegenständen, sowie die Verwendung der neuen urethangruppenhaltigen (Meth)Acrylate vorallem zur Herstellung von Beschichtungsmitteln, Klebstoffen, Photoresists und Lötstoppmasken.

Bekanntlich können strahlungsempfindliche Harze oder Harzgemische vielseitig angewendet werden, beispielsweise als Beschichtungsmittel, Klebstoffe oder Photoresists. Im Prinzip sollten diese Harze oder Harzsysteme generell auch für die Herstellung von dreidimensionalen Gegenständen (3D-Objekten) nach dem im US-Patent 4,575,330 beschriebenen stereolithographischen Verfahren geeignet sein, doch erweisen sich viele Harze als zu viskos, und andere wiederum sind zu wenig lichtempfindlich oder unterliegen beim Härten einem zu grossen Schrumpfungsprozess. Auch lassen die Festigkeitseigenschaften der Formkörper bzw. der Gegenstände aus photogehärteten Harzen oft zu wünschen übrig.

Mittels des stereolithographischen Verfahrens kann man bekanntlich komplizierte dreidimensionale Gegenstände aus flüssigen, lichtempfindlichen Harzen herstellen. Solche Gegenstände werden schichtweise aufgebaut, wobei jede neue härtbare Harzschicht durch Vorhärtung mittels UV/VIS-Licht mit der vorhergehenden, vorgehärteten Schicht fest verbunden wird. Der Gesamtaufbau des dreidimensionalen Gegenstandes lässt sich bekanntlich nach einem computergesteuerten Prozess bewerkstelligen.

In den letzten Jahren hat es nicht an Versuchen gefehlt, Harzsysteme zu entwickeln, welche in stereolithographischen Verfahren einsetzbar sind. H. Kodama offenbart in Rev. Sci. Instrum. **52** (11), 1770-1773, (1981) unter dem Handelsnamen "Tevista" ein flüssiges, photohärtbares Harzgemisch bestehend aus einem ungesättigten Polyester, einem Acrylsäureester, Styrol, einem Polymerisationsinitiator und einem Sensibilisator. Dieses Harzsystem ist für das stereolithographische Verfahren jedoch mit dem Nachteil behaftet, dass die Photoempfindlichkeit ungenügend ist und die sogenannte "Grünfestigkeit" (green strength) der mit Laserstrahlen vorgehärteten Gegenstände relativ niedrig ist.

Im US-Patent Nr. 4,575,330 wird ein stereolithographisches Verfahren vorgeschlagen, wonach als flüssiges Harz ein modifiziertes Acrylat eingesetzt wird, das in der Beschreibung als "Potting Compound 363" bezeichnet wird. Solche Harzgemische werden im US-Patent Nr. 4,100,141 offenbart. Auch sie haben den Nachteil, dass sie ungenügend photoempfindlich sind und für die Herstellung von dreidimensionalen Gegenständen nach dem stereolithographischen Verfahren lange Zeiten benötigen.

Daher ist es verständlich, dass an die Harze, die in stereolithographischen Verfahren eingesetzt werden sollen, sehr hohe Anforderungen gestellt werden. Beispielsweise soll die Photoempfindlichkeit des Harzsystems so beschaffen sein, dass das Verhältnis von aufgewendeter Strahlungsenergie und erzielter Eindringtiefe in das flüssige photoempfindliche Harzgemisch, wobei die betreffenden Teile sich verfestigen, im vertretbaren Rahmen liegt. Das heisst, bei einem für das stereolithographische Verfahren geeigneten Harz oder Harzgemisch soll mit wenig Strahlungsenergie eine möglichst hohe Härtungstiefe bei gleichzeitig hohem Polymerisationsgrad und guter Grünfestigkeit erreicht werden.

Beim Verfahren der aufeinanderfolgenden Polymerisation von dünnen Schichten, wie es im stereolithographischen Verfahren angewendet wird, ist gewöhnlich keine dieser Schichten völlig ausgehärtet. Der nicht völlig gehärtete Gegenstand wird als Grünling bezeichnet, und den Elastizitätsmodul und die Bruchfestigkeit dieses Grünlings bezeichnet man auch als Grünfestigkeit. Normalerweise wird der Grünling dann mit UV und/oder VIS-Licht gehärtet, beispielsweise mittels einer Quecksilber- oder Xenonbogenlampe. Die Grünfestigkeit eines Werkstückes stellt daher einen wichtigen Parameter dar, da Gegenstände mit einer geringen Grünfestigkeit sich unter ihrem eigenen Gewicht deformieren können, oder sie können sich bei der Aushärtung durchbiegen oder sacken zusammen.

Gemäss dem Stand der Technik werden, um brauchbare Verbindungen vorallem für die Stereolithographie zu haben, u.a. in der EP-A-0 360 869 Mischungen von Acrylatverbindungen mit Epoxidharzen vorgeschlagen. Diese haben jedoch den Nachteil, dass sie spröde Endprodukte ergeben. Ausserdem entstehen bei der Härtung zwei unabhängige Netzwerke, was sich nachteilig auf die Grünfestigkeit und die Endeigenschaften auswirkt.

Aus den JP-A 62-112666, JP-A 62-112667 und dem US-Patent Nr. 5,059,511 sind ausserdem urethangruppenhaltige Acrylat-Strukturen bekannt, die weitere polymerisierbare Gruppen aufweisen und die als Beschichtungsmaterialien bei der Herstellung von magnetischen Aufzeichnungsmedien eingesetzt werden können. Diese Acrylate können jedoch nur radikalisch polymerisiert werden, da es sich bei den weiteren polymerisierbaren Gruppen um Acrylate oder Allylverbindungen handelt.

Aufgabe der Erfindung war es daher, Verbindungen zu entwickeln, die die aufgezeigten Nachteile überwinden, d.h. für stereolithographische Anwendungen die richtige Viskosität und Lichtempfindlichkeit aufweisen, und welche kein unabhängiges Netzwerk, sondern ein zusammenhängendes, homogenes Netzwerk ergeben, so dass die Festigkeitseigenschaften der Formkörper und die mechanischen Eigenschaften generell verbessert werden können.

Gelöst wurde diese Aufgabe durch eine neue Klasse von polymerisierbaren urethangruppenhaltigen (Meth)Acrylaten. Diese enthalten in ein- und demselben Molekül sowohl mindestens eine (Meth)Acrylatgruppe und mindestens eine Urethangruppe sowie eine andere weitere polymerisierbare Gruppe und stellen demnach multifunktionelle Verbindungen dar.
Diese Verbindungen können erhalten werden, indem man HO-gruppenhaltige (Meth)Acrylate mit einem Diisocyanat und anschliessend mit einem Alkohol umgesetzt, der die gewünschten polymerisierbaren Gruppen enthält (bzw. aus dem die polymerisierbare Gruppe hergestellt werden kann). In den oben genannten JP-OS sind nur Umsetzungsprodukte aus HO-gruppenhaltigen Acrylaten mit einem Diisocyanat und einem acrylat- oder allylhaltigen Alkohol bekannt. Dies führt entweder zu vierfunktionellen Acrylaten (bei denen alle Acrylatgruppen nach dem gleichen Mechanismus und der gleichen Geschwindigkeit reagieren) oder zu difunktionellen Acrylaten mit zwei Allylgruppen. Diese Allylgruppen können jedoch nur thermisch, nicht aber mit einem Photoinitiator radikalisch polymerisiert werden.
Eine Klasse der neuen urethangruppenhaltigen (Meth)Acrylate enthält dagegen neben den (Meth)Acrylatgruppen noch kationisch polymerisierbare Gruppen, die nach einem anderen Mechanismus und mit anderer Geschwindingkeit reagieren. Eine andere Klasse enthält neben den (Meth)Acrylatgruppen weitere radikalisch polymerisierbare Gruppen, die entweder mit einem Photoinitiator radikalisch (aber mit anderer Geschwindigkeit als Acrylate) oder unter Zusatz von Thiolen und einem Photoinitiator in sog. Thiol-En-Systemen eine Polyaddition eingehen können.

Es ist überraschend, dass einerseits solche Moleküle relativ problemlos hergestellt werden können, dass diese leicht radikalisch und/oder kationisch polymerisieren und andererseits, dass diese Verbindungen z.B. in der Stereolithographie gut einsetzbar sind.

Durch Bestrahlung der aus diesen neuen polymerisierbaren urethangruppenhaltigen (Meth)Acrylaten hergestellten Zusammensetzungen können somit unterschiedlich hohe Vernetzungsdichten erreicht werden, was zur Folge hat, dass sich sowohl die bei der Vorhärtung mittels Laserstrahlen entstehenden Grünlinge als auch die durch Aushärtung der Grünlinge erhaltenen Gegenstände durch in weiten Grenzen variierbare und vorallem gute mechanische Eigenschaften, vorallem Festigkeitseigenschaften, auszeichnen.

Gegenstand vorliegender Erfindung sind somit neue urethangruppenhaltige (Meth)Acrylate, welche im Molekül (Meth)Acrylatgruppen und Urethangruppen sowie mindestens eine andere polymerisierbare Gruppe enthalten und somit ein Hybridsystem mit zwei verschiedenen funktionellen Gruppen, die nach verschiedenen Mechanismen polymerisiert werden können, darstellen. Es handelt sich dabei sowohl um offenkettige Strukturen als auch um Strukturen, die Ringelemente enthalten.

Die Erfindung betrifft polymerisierbare urethangruppenhaltige (Meth)Acrylate der Formel I 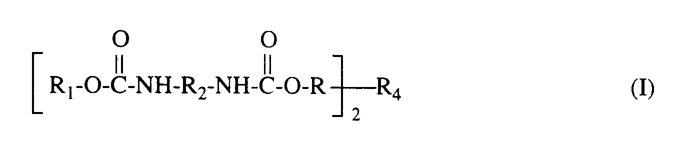 worin
- R: eine zweiwertige Gruppe der Formeln 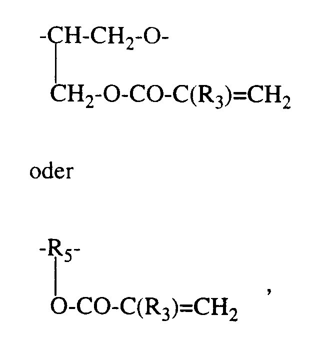
- R₁: eine kationisch polymerisierbare Gruppe der Formeln 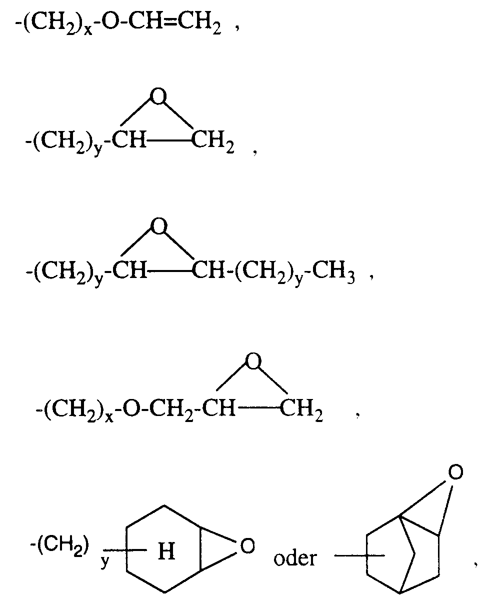 oder eine radikalisch polymerisierbare Gruppe der Formeln 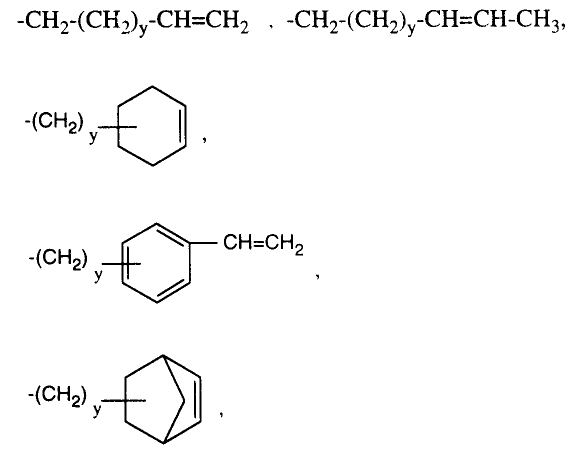 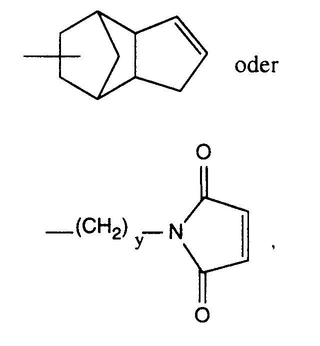 worin x eine ganze Zahl von 2 bis 20 und y eine ganze Zahl von 1 bis 20 bedeuten,
- R₂: einen aliphatischen, cycloaliphatischen oder aromatischen Rest,
- R₃: Wasserstoff oder CH₃,
- R₄: den Rest einer aliphatischen, cycloaliphatischen oder aromatischen Diglycidylverbindung nach Entfernen des Diglycidylrestes, oder den Rest eines cycloaliphatischen Diepoxides und
- R₅: ein cycloaliphatisches Brückenglied bedeuten.

Die Gruppe -(CH₂)ₓ- in diesen Formeln kann dabei ein- oder mehrmals unterbrochen sein, vorallem durch Arylen, wie Phenylen oder Naphthylen, und auch durch C₅- oder C₆-Cycloalkylengruppen, wie vorallem Cyclohexylen.

Bedeutet R eine Gruppe der Formel 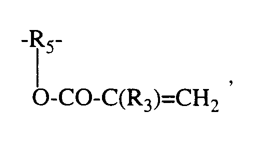 so befindet sich der Rest -O-CO-C(R₃)=CH₂ dieser Gruppe am Rest R₅, bevorzugt in o-Stellung zur -O-CO-NH-Bindung.

Besonders bevorzugte kationisch polymerisierbare Gruppen für R₁ entsprechen den Formeln

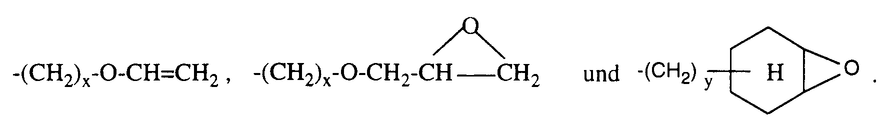

Bedeutet R₁ eine radikalisch polymerisierbare Gruppe, so kommen ebenfalls alle bekannten Gruppen, mit Ausnahme der Acrylat- und Allylgruppe, in Frage. Besonders bevorzugte radikalisch polymerisierbare Gruppen entsprechen dabei den Formeln

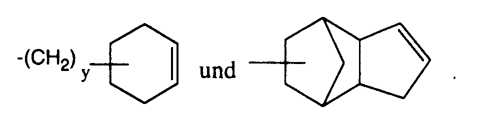

In all diesen Formeln bedeutet x eine ganze Zahl von 2 bis 20, vorzugsweise von 2 bis 6, und y ist eine ganze Zahl von 1 bis 20, vorzugsweise von 1 bis 6.

In den bevorzugten urethangruppenhaltigen (Meth)Acrylaten bedeutet R₁ eine kationisch polymerisierbare Gruppe.

Bedeuten R₂ einen aliphatischen, cycloaliphatischen oder aromatischen Rest und R₄ den Rest einer aliphatischen, cycloaliphatischen oder aromatischen Diglycidylverbindung oder eines cycloaliphatischen Diepoxides, so handelt es sich vorallem um ein C₄-C₂₀ aliphatisches Brückenglied, das durch mindestens eine Ether-, Ester-, Urethan-, Amid- oder Arylengruppe unterbrochen sein kann, oder um ein cycloaliphatisches oder aromatisches Brückenglied.

Bei den C₄-C₂₀ aliphatischen Brückengliedern handelt es sich sowohl um geradkettige wie verzweigte aliphatische Reste, wie Butylenreste, und um Pentylen-, Hexylen-, Heptylen-, Octylen-, Nonylen-, Decylen-, Undecylen-, Dodecylen-, Tetradecylen-, Hexadecylen-, Icosylen- oder Docosylen-reste.

Bei den cycloaliphatischen Brückengliedern R₂ und/oder R₄ handelt es sich vorallem um den Cyclohexylen- und Cyclopentylenrest.

Bei den aromatischen Brückengliedern R₂ und/oder R₄ handelt es sich vorallem um Arylen, wie Phenylen oder Naphthylen, oder um mehrere Arylengruppen, die durch aliphatische Reste unterbrochen sind, z.B. Diphenylmethan- oder Bisphenol-A- und -F-Reste nach Entfernen der Sauerstoffatome.

Sowohl die cycloaliphatischen Brückenglieder als auch die aromatischen Brückenglieder können ein- oder mehrmals substituiert sein, beispielsweise durch C₁-C₄-Alkyl (z.B. Methyl, Ethyl, n- und iso-Propyl, n-, sec.- und tert.- Butyl), C₁-C₄-Alkoxy (z.B. Methoxy oder Ethoxy) und Halogen (Fluor, Chlor, Brom oder Jod).

Bei R₂ handelt es sich z.B. um Brückenglieder der folgenden Formeln:

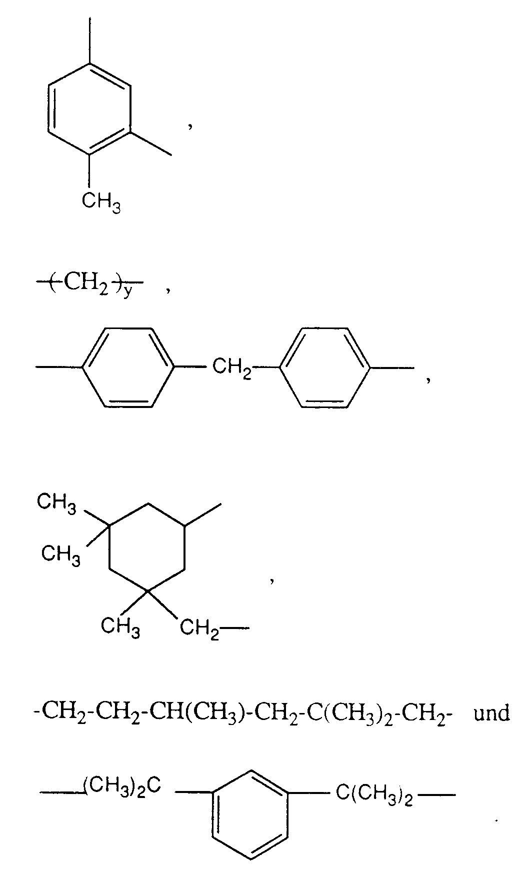

Als R₄ kommen z.B. Brückenglieder der folgenden Formeln in Frage: 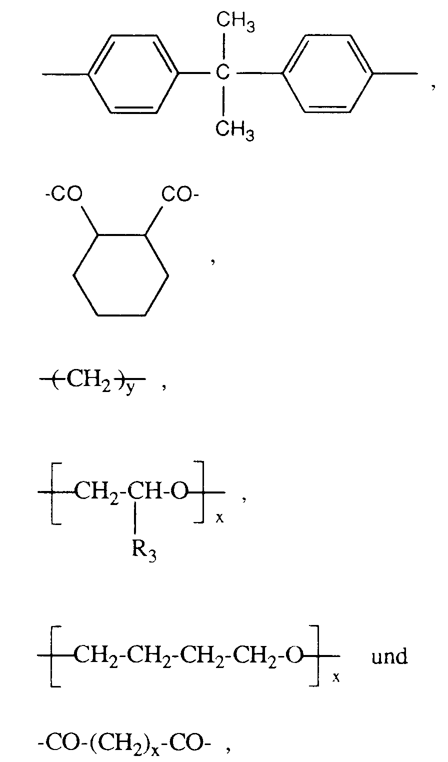 wobei x und y die oben angegebene Bedeutung haben.

In bevorzugten urethangruppenhaltigen (Meth)Acrylaten bedeutet R₂ ein C₄-C₂₀-aliphatisches oder ein aromatisches Brückenglied, oder mehrere aromatische Gruppen, die durch aliphatische Gruppen unterbrochen sind, und R₄ bedeutet ein C₄-C₂₀ aliphatisches Brückenglied, das durch mindestens eine Ether- oder eine Estergruppe unterbrochen ist, oder ein aromatisches Brückenglied.
Bedeuten R₂ und/oder R₄ ein aliphatisches Brückenglied, das durch Ether- oder Estergruppen unterbrochen ist, so kommen z.B. folgende Reste in Frage: 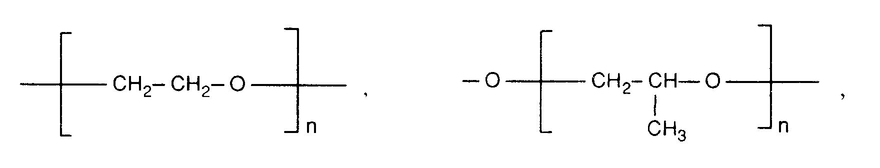 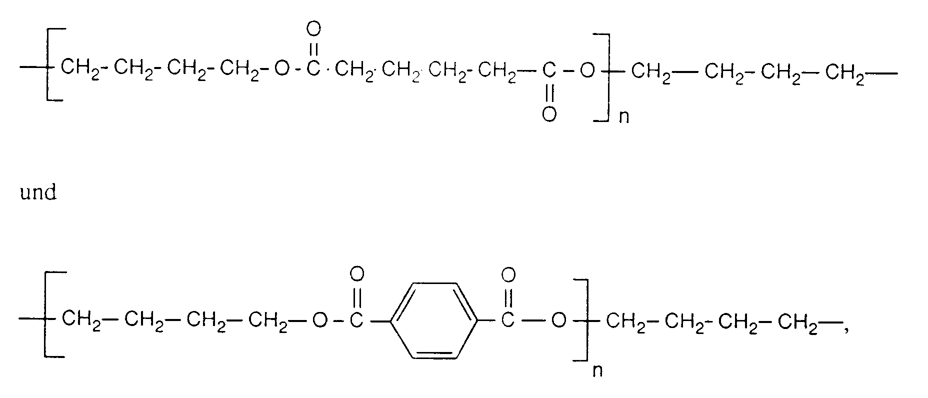 wobei n eine ganze Zahl von 1 bis 20 bedeutet.

Bei den cycloaliphatischen Brückengliedern R₅ handelt es sich z.B. um Cyclopentyl und Cyclohexyl.

Die erfindungsgemässen, neuen urethangruppenhaltigen (Meth)Acrylate sind nieder- bis hochviskose Harze, die sich leicht in organischen Lösungsmitteln, wie Toluol, Essigester und Tetrahydrofuran, lösen.

Die erfindungsgemässen, neuen urethangruppenhaltigen (Meth)Acrylate können in reinen Acrylatformulierungen eingesetzt werden. Sie erhöhen die Netzwerkdichte und damit den E-Modul. Ausserdem verlangsamen sie die Reaktion, durch welche SL-Formkörper mit geringerem (etwa 10-20%) Curl erhalten werden.

Es ist überraschend, dass die zweite polymerisierbare Gruppe ohne Probleme in das bestehende Diacrylat eingeführt werden kann. So werden die (Meth)Acrylatgruppen nicht zerstört, wenn die Epoxidgruppen eingeführt werden, und die sehr reaktiven Vinylether zeigen keine Nebenreaktionen mit den (Meth)Acrylaten. Die neuen epoxygruppenhaltigen Urethanacrylate konnten überraschenderweise in Hybridsystemen eingesetzt werden, obwohl aus der Literatur bekannt ist, dass Epoxide in Gegenwart von Urethangruppen nicht kationisch polymerisieren (S.C. Lapin, Polym. Mater. Sci. Eng. 61, 302 (1989)).

Die polymerisierbaren urethangruppenhaltigen (Meth)Acrylate können in an sich bekannter Art und Weise hergestellt werden, z.B. erfolgt die Herstellung dadurch, dass ein Epoxyacrylat der Formel II

[ HO-R⁆₂R₄ (II)

mit einem Diisocyanat der Formel III

OCN-R₂-NCO (III)

zweckmässig in äquimolaren Mengen in einem organischen Lösungsmittel in Gegenwart eines Katalysators und eines Inhibitors zur Reaktion gebracht werden, und die entstandene Verbindung der Formel IV

[ OCN-R₂-NH-CO-O-R⁆₂R₄ (IV)

mit einer den Rest R₁ einführenden Verbindung umgesetzt wird. Gegebenenfalls können dann die so erhaltenen polymerisierbaren urethangruppenhaltigen (Meth)Acrylate der Formel I noch oxydiert werden. R_{1,} R₂ und R₄ haben die oben bereits angegebenen Bedeutungen.

Als Epoxyacrylate der Formel II kommen alle bekannten Arten in Frage, wie z.B. Bisphenol-A-diglycidyldiacrylat, Butandioldiglycidyldiacrylat, Bisphenol-F-diglycidyldiacrylat und Polypropylenglykoldiglycidyldiacrylat, ferner Umsetzungsprodukte von Acrylsäure mit cycloaliphatischen Epoxidharzen.

Die Diisocyanate der Formel III sind ebenfalls bekannt und können auf bekannte Art und Weise hergestellt werden. Genannt sind z.B. aliphatische, cycloaliphatische und aromatische Diisocyanate, wie Hexamethylendiisocyanat, Trimethylhexamethylendiisocyanat, Cyclohexandiisocyanat, Isophorondiisocyanat(3,5,5-Trimethyl-1-isocyanato-3-isocyanatomethylcyclohexan), Methylendicyclohexyldiisocyanat, p-Phenylendiisocyanat, 2,4-Diisocyanatotoluol, 2,6-Diisocyantatotoluol und technische Gemische von beiden Isomeren, Naphthylendiisocyanate, insbesondere 1,5-Naphthylendiisocyanat, Dianisidindiisocyanat, Methylendiphenyldiisocyanate, insbesondere das 4,4 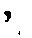 -Isomere, aber auch technische Gemische verschiedener Isomeren, beispielsweise der 4,4 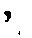 - und 2,4 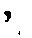 -Isomeren, oder Polymethylenpolyphenylendiisocyanate; besonders bevorzugt ist Toluylendiisocyanat.

Die Reaktion des Epoxyacrylates der Formel II mit dem Diisocyanat der Formel III wird zweckmässig in einem organischen Lösungsmittel in Gegenwart eines Katalysators und eines Inhibitors bei einer Temperatur von etwa 30-40°C, vorzugsweise bei 35°C, durchgeführt.

Als organische Lösungsmittel kommen z.B. in Frage: aromatische Lösungsmittel, wie Toluol und Xylole, sowie aliphatische Lösungsmittel, wie Chloroform, Methylenchlorid und Ethylacetat.

Als Katalysatoren können z.B. eingesetzt werden: Dibutylzinndilaurat oder Zinnoctoat.

Inhibitoren sind z.B. 2,2 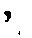 -Methylen-bis-(6-tert.-butyl-4-methyl-phenol) (= Ralox® 46), p-Methoxyphenol und Di-tert.-butyl-p-kresol.

Die erhaltenen Reaktionsprodukte der Formel IV sind bekannt. Sie werden ohne Isolation d.h. in einem einstufigen Verfahren, mit einer den Rest R₁ einführenden Verbindung zu den urethangruppenhaltigen (Meth)Acrylaten der Formel I umgesetzt.

Als den Rest R₁ einführende Verbindungen kommen solche in Frage, die mindestens eine Hydroxylgruppe aufweisen. Geeignet sind z.B. Alkohole, wie Tetrahydrobenzylalkohol und Crotylalkohol, und die entsprechenden Alkohole der oben als R₁ aufgeführten Reste.

Die Umsetzung der Verbindung der Formel IV mit der den Rest R₁ einführenden Verbindung erfolgt vorteilhaft im gleichen Lösungsmittel und bei der gleichen Temperatur wie für die Reaktion des Epoxyacrylates der Formel II mit dem Diisocyanat der Formel III. Anschliessend wird das organische Lösungsmittel abdestilliert; zurück bleibt ein weissgelbes bis gelbes Harz, welches dem polymerisierbaren urethangruppenhaltigen (Meth)Acrylat der Formel I entspricht.

Dieses Harz kann dann weiter oxydiert werden. Die Oxydation erfolgt z.B. mit einer Lösung von Peressigsäure in Essigsäure in Anwesenheit eines organischen Lösungsmittels, wie Chloroform, Methylenchlorid oder Ethylacetat, bei einer Temperatur von 15°C bis maximal 40°C.

Die erfindungsgemässen, neuen urethangruppenhaltigen (Meth)Acrylate können mit den verschiedensten Komponenten zur Herstellung von Zusammensetzungen weiterverarbeitet werden. Derartige Zusammensetzungen enthalten
a) 5 bis 99 Gew.% eines monomeren polymerisierbaren urethangruppenhaltigen (Meth)Acrylates der Formel I, und
b) 1 bis 10 Gew.% eines radikalischen Photoinitiators.

Typische Verbindungen bekannter Photoinitiatoren sind Benzoine, wie Benzoin, Benzoinether, wie Benzoinmethylether, Benzoinethylether, Benzoinisopropylether und Benzoinphenylether, Benzoinacetat, Acetophenone, wie Acetophenon, 2,2-Dimethoxyacetophenon und 1,1-Dichloracetophenon, Benzil, Benzilketale, wie Benzildimethylketal und Benzildiethylketal, Anthrachinone, wie 2-Methylanthrachinon, 2-Ethylanthrachinon, 2-tert.-Butylanthrachinon, 1-Chloranthrachinon und 2-Amylanthrachinon, ferner Triphenylphosphin, Benzoylphosphinoxide, wie beispielsweise 2,4,6-Trimethylbenzoyldiphenylphosphinoxid (Luzirin TPO), Benzophenone, wie Benzophenon und 4,4'-Bis-(N,N'-dimethylamino)benzophenon,Thioxanthone und Xanthone, Acridinderivate, Phenazinderivate, Quinoxalinderivate oder 1-Phenyl-1,2-propandion-2-O-benzoyloxim, 1-Aminophenylketone oder 1-Hydroxyphenylketone, wie 1-Hydroxycyclohexylphenylketon, Phenyl-(1-hydroxyisopropyl)-keton und 4-Isopropylphenyl-(1-hydroxyisopropyl)-keton, welche alle bekannte Verbindungen darstellen.

Besonders geeignete Photoinitiatoren, welche gewöhnlich in Kombination mit einem He/Cd-Laser als Lichtquelle verwendet werden, sind Acetophenone, wie 2,2-Dialkoxybenzophenone und 1-Hydroxyphenylketone, beispielsweise 1-Hydroxycyclohexylphenylketon oder 2-Hydroxyisopropylphenylketon (= 2-Hydroxy-2,2-dimethylacetophenon).

Eine andere Klasse von Photoinitiatoren, die gewöhnlich bei Verwendung von Argonion-Lasern eingesetzt wird, sind die Benzilketale, wie beispielsweise Benzildimethylketal.

Eine weitere Klasse von geeigneten Photoinitiatoren stellen die ionischen Farbstoff-Gegenionverbindungen (ionic dye-counter ion compound) dar, welche in der Lage sind, aktinische Strahlen zu absorbieren und freie Radikale zu erzeugen, die die Polymerisation der Epoxidverbindungen initiieren können. Die Zusammensetzungen, welche ionische Farbstoff-Gegenionverbindungen enthalten, können auf diese Weise variabler mit sichtbarem Licht im einstellbaren Wellenlängenbereich von 400-700 nm gehärtet werden. Ionische Farbstoff-Gegenionverbindungen und deren Wirkungsweise sind bekannt, beispielsweise aus der EP-Patentanmeldung Nr. 223 587 und den US-Patenten Nr. 4,751,102; 4,772,530 und 4,772,541. Als Beispiele für geeignete ionische Farbstoff-Gegenionverbindungen sind genannt: die anionischen Farbstoff-Jodoniumionkomplexe, die anionischen Farbstoff-Pyrylliumionkomplexe und insbesondere die kationischen Farbstoff-Boratanionverbindungen der folgenden Formel 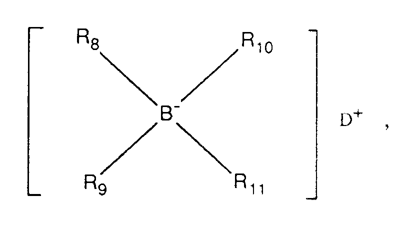 worin D⁺ für einen kationischen Farbstoff steht und R₈, R₉, R₁₀ und R₁₁ unabhängig voneinander je ein Alkyl, Aryl, Alkaryl, Allyl, Aralkyl, Alkenyl, Alkinyl, eine alicyclische oder gesättigte oder ungesättigte heterocyclische Gruppe bedeuten. Bevorzugte Definitionen für die Reste R₈ bis R₁₁ können beispielsweise der oben genannten EP-Patentanmeldung 223 587 entnommen werden.

Die Photoinitiatoren werden bekanntlich in wirksamen Mengen zugesetzt, das heisst, zweckmässig in Mengen von 2 bis 10 Gewichtsprozenten, bezogen auf die Gesamtmenge der Zusammensetzung. Wenn die erfindungsgemässen Zusammensetzungen für stereolithographische Verfahren verwendet werden, nach welchen normalerweise Laserstrahlen eingesetzt werden, ist es wesentlich, dass die Absorptionsfähigkeit der Zusammensetzung durch Typ und Konzentration des Photoinitiators so abgestimmt wird, dass die Härtungstiefe bei normaler Lasergeschwindigkeit ungefähr 0,1 bis 2,5 mm beträgt.

Weitere geeignete Photoinitiatoren können auch Verbindungen sein, welche gegenüber Strahlen von Emissionslinien unterschiedlicher Wellenlängen verschieden strahlungsempfindlich sind. Man erreicht dadurch beispielsweise eine bessere Ausnützung einer UV und/oder VIS-Lichtquelle, welche Emissionslinien unterschiedlicher Wellenlänge ausstrahlt. Vorteilhaft ist es dabei, wenn die verschiedenen Photoinitiatoren so ausgewählt und in einer solchen Konzentration eingesetzt werden, dass bei den verwendeten Emissionslinien eine gleiche optische Absorption erzeugt wird.

Bevorzugte Photoinitiatoren sind 1-Hydroxyphenylketone, insbesondere 1-

Diese Zusammensetzungen enthalten weiter:
c) 0 bis 20 Gew.% üblicher Additive, beispielsweise Stabilisatoren, wie UV-Stabilisatoren, Polymerisationsinhibitoren, Trennmittel, Benetzungsmittel, Verlaufsmittel, Sensibilisatoren, Antiabsetzmittel, oberflächenaktive Mittel, Farbstoffe, Pigmente oder Füllstoffe;
d) 0 bis 80 Gew.% eines oder mehrerer mono-, di- oder mehrfunktionellen (Meth)Acrylate, wie Mono(meth)acrylat, Mono-N-Vinylverbindungen mit einem Molekulargewicht von höchstens 500, aliphatische oder cycloaliphatische Di(meth)acrylate, aliphatische Tri(meth)acrylate oder aromatische Di- oder Tri(meth)acrylate, oder Mischungen davon;
e) 0 bis 80 Gew.% eines oder mehrerer di- oder mehrfunktionellen üblichen aromatischen, alicyclischen oder aliphatischen Epoxidharze oder Gemische davon; Epoxidharze, die in der erfindungsgemässen Formulierung in Frage kommen, sind z.B. in der EP-A-0 360 869 beschrieben. Vorzugsweise werden Butandioldiglycidylether und 3,4-Epoxycyclohexyl-3 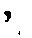 ,4 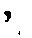 -epoxycyclohexancarboxylat eingesetzt;
f) 0 bis 50 Gew.% eines OH-terminierten Polyethers oder Polyesters, wie di-oder trifunktionelle Polyether- oder Polyester-Polyole, Polytetrahydrofuran, Poly-ε-caprolacton und OH-terminierte Polyurethane oder Gemische davon. Von besonderem Interesse ist OH-terminiertes Polycaprolacton;
g) 0 bis 5 Gew.% eines kationischen Photoinitiators, wie sie in der EP-A-0 360 869 beschrieben sind;
   Von besonderem Interesse sind Triarylhexafluoroantimonat, wie Triarylsulfonium-hexafluoroantimonat.
h) 0-80 Gew.% eines oder mehrerer mono-, di- oder mehrfunktionellen Vinylether, wie in EP 360869 beschrieben.
   Bevorzugte Zusammensetzungen enthalten
   a) 10 bis 60 Gew.% eines monomeren polymerisierbaren urethangruppenhaltigen (Meth)Acrylates der Formel I,
   b) 0,5 bis 7 Gew.% eines radikalischen Photoinitiators,
   c) 0 bis 10 Gew.% üblicher Additive,
   d) 10 bis 70 Gew.% eines oder mehrerer mono-, di- oder mehrfunktionellen (Meth)Acrylate,
   e) 0 bis 60 Gew.% eines oder mehrerer di- oder mehrfunktionellen Epoxide,
   f) 0 bis 50 Gew.% eines OH-terminierten Polyethers oder Polyesters oder Gemische davon,
   g) 0,5 bis 5 Gew.% eines kationischen Photoinitiators, und
   h) 0 bis 60 Gew.% eines oder mehrerer di- oder mehrfunktionellen Vinylether.

Die erfindungsgemässen Zusammensetzungen, die eine hohe Photoempfindlichkeit aufweisen, können nach bekannter Art und Weise hergestellt werden, beispielsweise durch Vormischen einzelner Komponenten und anschliessendes Vermischen dieser Vormischungen, oder durch Vermischen aller Komponenten mittels üblicher Vorrichtungen, wie Rührbehälter, zweckmässig in Abwesenheit von Licht und gegebenenfalls bei leicht erhöhter Temperatur, z.B. bei etwa 50 bis 70°C.

Die erfindungsgemässen Zusammensetzungen, die zusammenhängende Netzwerke enthalten, sind Flüssigkeiten mit einer Viskosität von etwa 100 bis 5000 mPas bei 30°C, vorzugsweise von 200 bis 4500 mPas und insbesondere von 200 bis 2000 mPas. Diese Zusammensetzungen weisen bei hoher Lichtempfindlichkeit überraschenderweise einen niedrigen Schrumpfungsfaktor (curl factor) sowohl in Hybridsystemen als auch in Acrylatsystemen auf, besitzen eine hohe Härtungstiefe und eine gute Grünfestigkeit, und die daraus hergestellten Formkörper haben ausgezeichnete mechanische Festigkeitseigenschaften.

Die erfindungsgemäss in Frage kommenden Zusammensetzungen, enthaltend die neuen polymerisierbaren urethangruppenhaltigen (Meth)Acrylate, können durch Bestrahlen mit aktinischem Licht, beispielsweise mittels Elektronen- oder Röntgenstrahlen, ferner UV-oder VIS-Licht, das heisst, mit elektromagnetischer Strahlung oder Teilchenstrahlung im Wellenlängenbereich von 280-650 nm, polymerisiert werden. Besonders geeignet sind Laserstrahlen von He/Cd, Argon- oder Stickstoff- sowie Metalldampf- und NdYAG-Laser mit vervielfachter Frequenz. Es ist dem Fachmann bekannt, dass für jede gewählte Lichtquelle der geeignete Photoinitiator ausgewählt und gegebenenfalls sensibilisiert werden muss. Man hat erkannt, dass die Eindringtiefe der Strahlung in die zu polymerisierende Zusammensetzung und die Arbeitsgeschwindigkeit in direktem Zusammenhang mit dem Absorptionskoeffizienten und der Konzentration des Photoinitiators stehen. In der Stereolithographie werden vorzugsweise solche Photoinitiatoren eingesetzt, welche die grösste Strahlungseindringtiefe in die zu polymerisierenden Zusammensetzungen ermöglichen.

Gegenstand der Erfindung ist somit auch ein Verfahren zum Polymerisieren der erfindungsgemässen Zusammensetzungen, indem man diese mit aktinischem Licht bestrahlt. Die erhaltenen Polymerisate können beispielsweise als Beschichtungsmittel, Photoresists, Lötstoppmasken oder Klebstoffe verwendet werden.

Werden die erfindungsgemäss in Frage kommenden Zusammensetzungen als Beschichtungsmittel eingesetzt, so erhält man klare und harte Beschichtungen z.B. auf Holz, Papier, Metall, Keramik oder anderen Oberflächen. Die Beschichtungsdicke kann dabei sehr variieren und etwa von 1 µm bis etwa 1 mm betragen. Aus den erfindungsgemässen Zusammensetzungen können Reliefbilder für gedruckte Schaltungen oder Druckplatten direkt durch Bestrahlen der Gemische hergestellt werden, beispielsweise mittels eines computergesteuerten Laserstrahls geeigneter Wellenlänge oder unter Anwendung einer Photomaske und einer entsprechenden Lichtquelle.

Eine weitere Verwendungsmöglichkeit ist der Einsatz der erfindungsgemässen Zusammensetzungen als photohärtbare Klebstoffe.

Vorzugsweise verwendet man die erfindungsgemässen Zusammensetzunge zur Herstellung von photopolymerisierten Schichten, insbesondere in Form von dreidimensionalen Gegenständen, die aus mehreren aneinander haftenden, verfestigten Schichten aufgebaut sind.

Ein weiterer Erfindungsgegenstand ist demnach auch ein Verfahren zur Herstellung von dreidimensionalen Gegenständen aus einer erfindungsgemäss in Frage kommenden Zusammensetzung mittels einem lithographischen Verfahren, insbesondere stereolithographischen Verfahren, wobei die Oberfläche einer Schicht aus der erfindungsgemässen Zusammensetzung ganzflächig oder in einem vorbestimmten Muster mit einer UV-und/oder VIS-Lichtquelle bestrahlt wird, so dass sich in den bestrahlten Bereichen eine Schicht in einer gewünschten Schichtdicke verfestigt, dann eine neue Schicht aus der Zusammensetzung auf der verfestigten Schicht gebildet wird, die ebenfalls ganzflächig oder in einem vorbestimmten Muster bestrahlt wird, und wobei durch wiederholtes Beschichten und Bestrahlen dreidimensionale Gegenstände aus mehreren aneinander haftenden, verfestigten Schichten erhalten werden.

Bei diesem Verfahren wird als Lichtquelle vorzugsweise ein Laserstrahl verwendet, der in einer besonders bevorzugten Ausführungsform computergesteuert ist.

Die folgenden Beispiele veranschaulichen die vorliegende Erfindung weiter, ohne sie darauf zu beschränken.

### Beispiel 1:

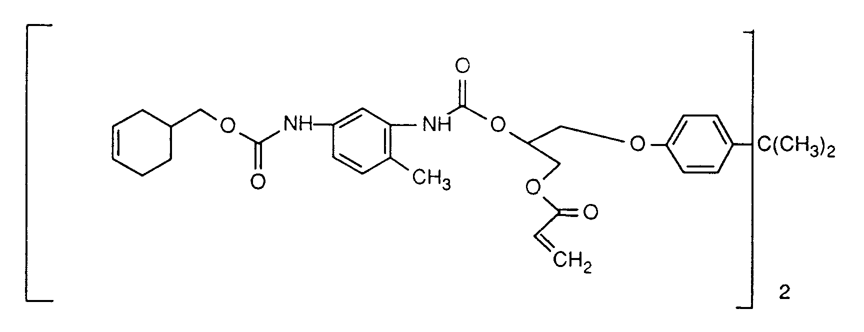

71,88 g (0,41 mol) Toluylendiisocyanat, 0,52 g Dibutylzinndilaurat und 0,2 g Ralox® 46 werden in einem Reaktionsgefäss unter Einleitung eines Luftstroms auf 35°C erwärmt. 100 g (0,206 mol) Bisphenol-A-diglycidyldiacrylat (Novacure 3700, UCB), gelöst in 200 ml Toluol, werden langsam zugetropft, wobei eine leichte Exothermie zu beobachten ist. Die erhaltene Mischung wird ca. 5 Stunden bei 35°C gerührt, bis der Isocyanatgehalt auf 1,12 Aequ./kg abgesunken ist. Dann werden langsam 46,2 g (0,412 mol) Tetrahydrobenzylalkohol zugetropft. Die Reaktion ist leicht exotherm. Die Mischung wird so lange bei 35°C gerührt, bis der Isocyanatgehalt <0,02 Aequ./kg beträgt (ca. 11 Stunden). Nach Abdestillieren des Lösungsmittels im Hochvakuum (HV) wird ein viskoses, gelbes Harz der oben angegebenen Stuktur erhalten.
GPC (Gelpermeationschromatographie): Mn = 1390, Mw = 2090

### Beispiel 2:

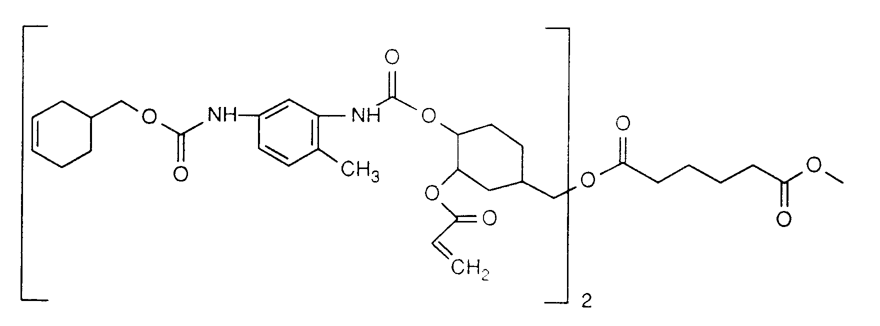

136,44 g (0,78 mol) Toluylendiisocyanat, 1,48 g Dibutylzinndilaurat und 1,02 g Ralox® 46 werden in einem Reaktionsgefäss unter Einleitung eines Luftstroms auf 35°C erwärmt. 200 g (0,38 mol) des Umsetzungsproduktes aus Acrylsäure und dem cycloaliphatischen Epoxidharz

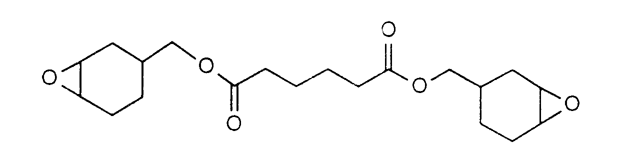

(Araldit CY 177, Ciba-Geigy), gelöst in 400 ml Chloroform, werden langsam zugetropft. Die exotherme Reaktion wird mit einem Eisbad auf 35°C gehalten. Nach ca. 2,5 Stunden wird ein Isocyanatgehalt von 0,75 Aequ./kg erreicht. Dann werden 87,48 g (0,78 mol) Tetrahydrobenzylalkohol zugetropft und so lange gerührt, bis der Isocyanatgehalt auf <0,08 Aequ./kg abgesunken ist (ca. 2 Stunden). Das Lösungsmittel wird im HV abdestilliert. Das erhaltene Produkt weist die oben angegebene Struktur auf.

### Beispiel 3:

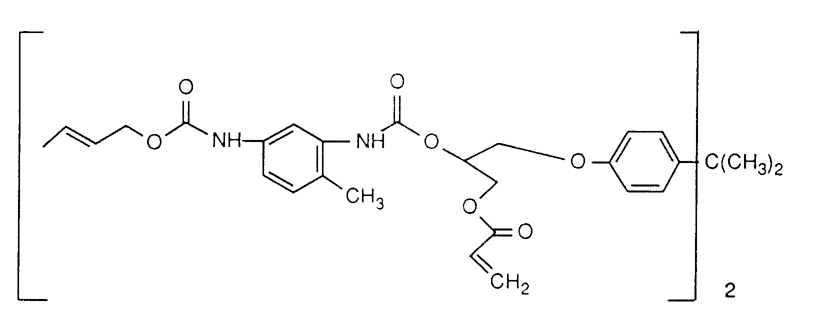

81,9 g (0,41 mol) Toluylendiisocyanat, 0,52 g Dibutylzinndilaurat und 0,52 g Ralox® 46 werden in einem Reaktionsgefäss auf 35°C erhitzt. 100 g (0,206 mol) Bisphenol-A-diglycidyldiacrylat (Novacure 3700), gelöst in 100 ml Toluol, werden langsam zugetropft. Die Mischung wird mit einem Eisbad auf 35°C gehalten. Nach ca. 2 Stunden wird ein Isocyanatgehalt von 1,7 Aequ./kg erreicht. Dann werden 29,71 g (0,41 mol) Crotylalkohol zugetropft. Die Lösung wird so lange bei 35°C gerührt, bis der Isocyanatgehalt auf <0,08 Aequ./kg abgesunken ist. Das Lösungsmittel wird im HV abdestilliert. Das erhaltene Produkt hat die oben angegebene Struktur.

### Beispiel 4:

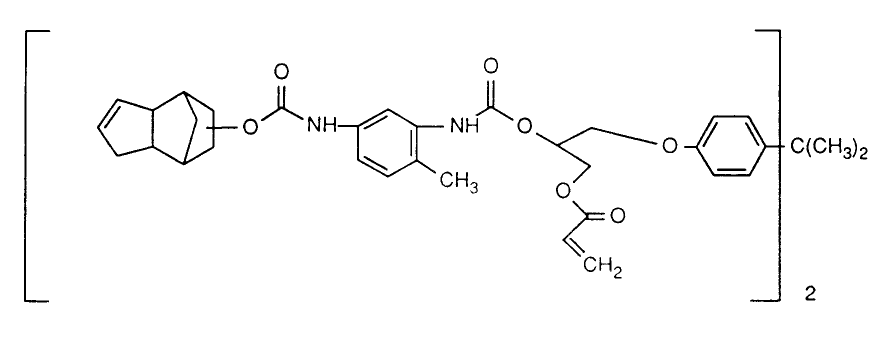

71,9 g (0,206 mol) Toluylendiisocyanat, 0,52 g Dibutylzinndilaurat und 0,52 g Ralox® 46 werden in einem Reaktionsgefäss auf 35°C erwärmt. 100 g (0,206 ml) Novacure 3700, gelöst in 100 ml Toluol, werden langsam zugetropft, so dass die Temperatur bei 35°C gehalten werden kann. Die Mischung wird so lange gerührt, bis ein Isocyanatgehalt von 1,6 Aequ./kg erhalten wird (ca. 5 Stunden). Dann werden 61,90 g (0,412 mol) TCD-Alkohol E [8(9)-Hydroxy-tricyclo[5.2.1.0^{2,6}]dec-3-en; Hoechst] zugetropft und so lange bei 35°C gerührt, bis der Isocyanatgehalt auf <0,1 Aequ./kg abgesunken ist (ca. 12 Stunden). Das Lösungsmittel wird im HV abdestilliert. Das erhaltene Produkt entspricht der oben angegebenen Struktur.
GPC: Mn = 1430, Mw = 265

### Beispiel 5:

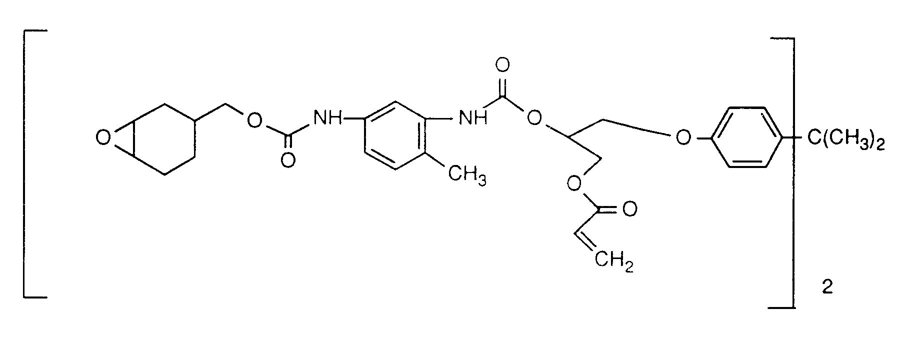

138,4 g (0,13 mol) des Produkts gemäss Beispiel 1, 10 g Natriumacetat und 0,65 g Hydrochinonmonomethylether werden in 200 ml Chloroform suspendiert. 68,8 g (0,37 mol) 40 % Peressigsäure werden langsam zugetropft, wobei die Temperatur nicht über 40°C steigen darf. Das Reaktionsgemisch wird noch 4 Stunden bei 35°C gerührt. Dann wird mit 5 % wässrigem NaHCO₃, und 2-mal mit Wasser extrahiert. Nach dem Trocknen der organischen Phase mit MgSO₄ und Zerstören der restlichen Peroxide mit NaHSO₃ wird das Lösungsmittel im HV abdestilliert. Das Produkt weist die oben angegebene Struktur auf.
GPC: Mn = 1370, Mw = 2590
Epoxidgehalt: 1,84 Aequ./kg (60 % der Theorie)

### Beispiel 6:

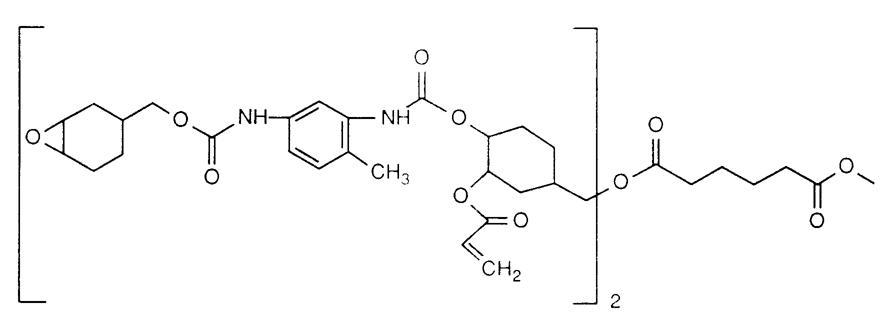

209 g (0,19 mol) des Umsetzungsprodukts gemäss Beispiel 2, 15 g Natriumacetat und 1 g Hydrochinonmonomethylether werden in ca. 500 ml Chloroform suspendiert. 101,1 g (0,53 mol) 40 % Peressigsäure werden langsam zugetropft, wobei die Temperatur nicht über 35°C steigen darf. Dann wird noch 4 Stunden bei 35°C weitergerührt. Das Gemisch wird mit 5 % NaHCO₃ und 2-mal mit Wasser extrahiert, die organische Phase wird getrocknet und nach Entfernen der restlichen Peroxide im HV eingeengt.
GPC: Mn 1020, Mw = 2240
Epoxidgehalt: 1,12 Aequ./kg (61,8 % der Theorie); chemische Formel: siehe oben.

### Beispiel 7:

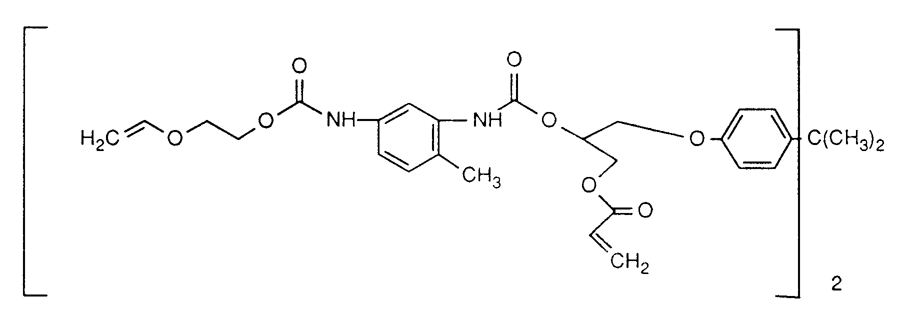

71,9 g (0,412 mol) Toluylendiisocyanat, 0,52 g Dibutylzinndilaurat und 0,52 g Ralox® 46 werden in einem Reaktionsgefäss unter Rühren auf 35°C erwärmt. 100 g (0,206 mol) Novacure 3700, gelöst in 100 ml Toluol, werden langsam zugetropft. Das Gemisch wird noch ca. 2 Stunden bei 35°C gerührt, bis ein Isocyanatgehalt von 1,6 Aequ./kg erreicht ist. Dann wird 36,3 g (0,42 mol) Hydroxyethylvinylether zugetropft. Nach weiteren 6 Stunden bei 35°C wird ein Isocyanatgehalt von <0,02 Aequ./kg erreicht. Das Lösungsmittel wird im HV abdestilliert. Das erhaltene Produkt weist die oben angegebene Struktur auf.
GPC: Mn = 1970, Mw = 5300
Gehalt an Doppelbindungen: 3,1 Aequ./kg (78 % der Theorie)

### Beispiel 8:

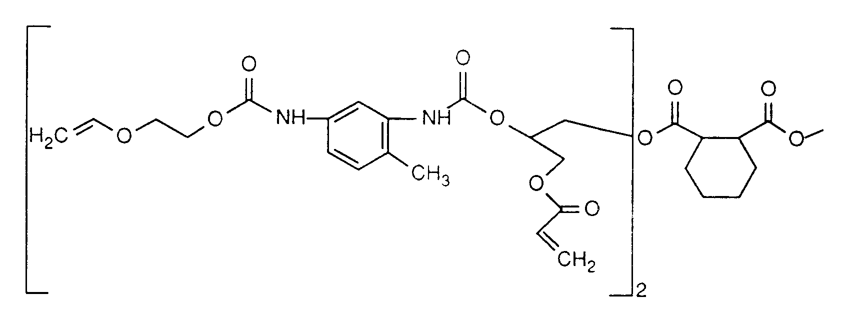

71,75 g (0,412 mol) Toluylendiisocyanat, 0,52 g Dibutylzinndilaurat und 0,48 g Ralox® 46 werden in einem Reaktionsgefäss unter Rühren auf 35°C erwärmt. 88,25 g (0,206 mol) des Reaktionsprodukts aus Hexahydrophthalsäurediglycidylester und Acrylsäure, gelöst in 100 ml Toluol, werden langsam zugetropft. Das Gemisch wird bei 35°C gerührt, bis ein Isocyanatgehalt von 1,0 Aequ./kg erreicht ist. Dann werden 36,3 g (0,412 mol) Hydroxyethylvinylether zugetropft. Nach weiteren 2 Stunden bei 35°C wird ein Isocyanatgehalt von <0,02 Aequ./kg erreicht. Das Lösungsmittel wird in HV abdestilliert. Das erhaltene Produkt weist die oben angegebene Struktur auf.
GPC: Mn = 1900, Mw = 6600

### Beispiel 9:

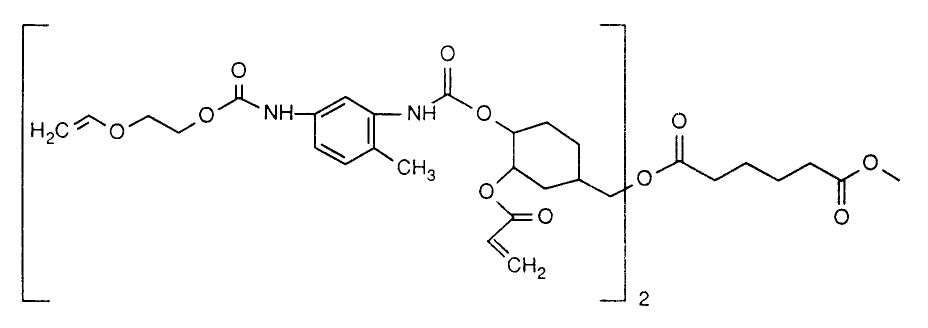

71,75 g (0,412 mol) Toluylendiisocyanat, 0,52 g Dibutylzinndilaurat und 0,53 g Ralox® 46 werden unter Rühren auf 35°C erwärmt. 105,18 g (0,206 mol) des Reaktionsprodukts aus 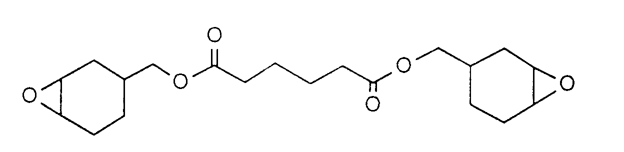 und Acrylsäure, gelöst in 100 ml Toluol, werden zugetropft. Nach ca. 5 Stunden bei 35°C ist ein Isocyanatgehalt von 1,1 Aequ./kg erreicht. Dann werden 36,3 g (0,412 mol) Hydroxyethylvinylether zugetropft. Nach ca. 2 Stunden bei 35°C ist ein Isocyanatgehalt von <0,05 Aequ./kg erreicht. Das Lösungsmittel wird im HV abdestilliert. Das erhaltene Produkt weist die oben angegebene Struktur auf.
GPC: Mn = 1100, Mw = 2100

### Beispiel 10:

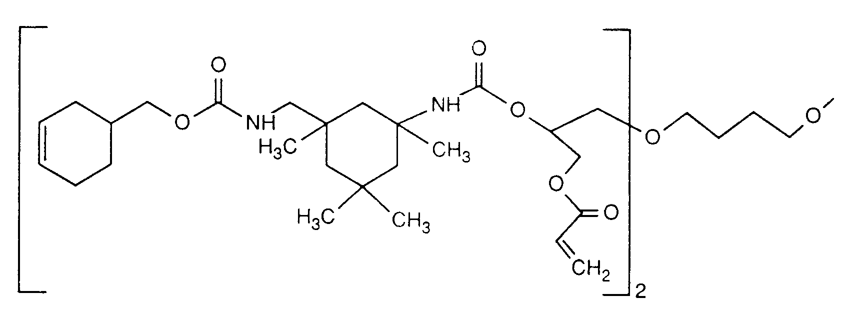

100 g (0,29 mol) Isophorondiisocyanat und 0,58 g Benzoylchlorid werden unter Rühren auf 35°C erhitzt. 65,06 g (0,58 mol) Tetrahydrobenzylalkohol werden zugetropft und bei 35°C gerührt, bis ein Isocyanatgehalt von 3,13 Aequ./kg erhalten wird (ca. 8 Stunden). Dann werden 100 g (0,29 mol) Laromer 8765 (Butandioldiglycidylether-diacrylat), gelöst in 100 ml Toluol, zugetropft. Die Mischung wird 34 Stunden bei 35°C gerührt, bis ein Isocyanatgehalt von 0,09 Aequ./kg erhalten wird. Das Lösungsmittel wird im HV abdestilliert. Es wird ein sehr hochviskoses, zähes Harz der oben angegebenen Struktur erhalten.
GPC: Mn = 1000, Mw = 1840

### Beispiel 11:

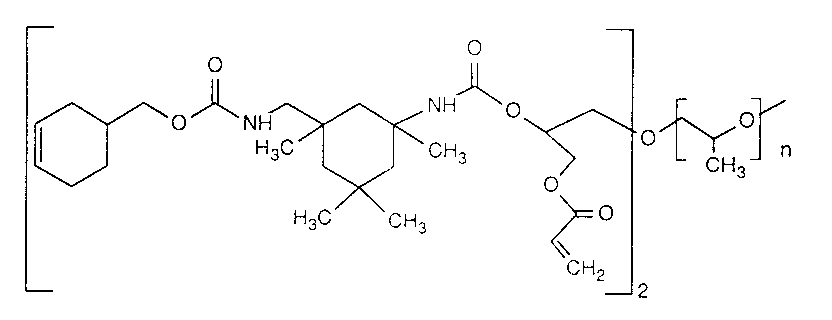

n = ca. 15

44,46 g (0,2 mol) Isophorondiisocyanat werden mit 0,2 g Benzoylchlorid auf 35°C erhitzt und 22,43 g (0,2 mol) Tetrahydrobenzylalkohol unter Rühren zugetropft. Die Mischung wird bei 35°C gerührt, bis ein Isocyanatgehalt von 3,05 Aequ./kg erhalten wird (ca. 13 Stunden). Dann werden 100 g (0,1 mol) Polypropylenglycol-400-diglycidyletherdiacrylat, gelöst in 100 ml Toluol, zugetropft. Die Lösung wird gerührt, bis ein Isocyanatgehalt von 0,13 Aequ./kg erhalten wird (ca. 38 Stunden). Das Lösungsmittel wird im HV entfernt.Das erhaltene Produkt weist die oben angegebene Struktur auf.
GPC: Mn = 1280, Mw = 2360

### Beispiel 12:

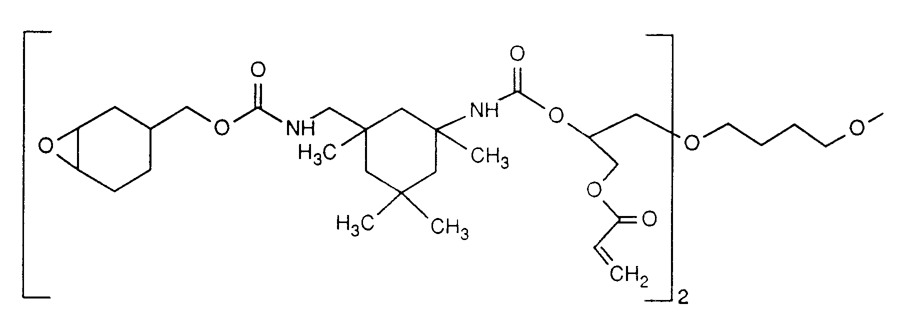

100 g (0,098 mol) des Produktes gemäss Beispiel 10 werden in 200 ml Chloroform gelöst und auf 35°C erhitzt. 10 g Natriumacetat und 0,49 g Hydrochinonmonomethylether werden zugefügt. Dann werden 53,24 g (0,28 mol) 40 % Peressigsäure langsam zugetropft. Die Lösung wird noch 6 Stunden bei 35°C gerührt und wie in Beispiel 5 aufgearbeitet. Das erhaltene Produkt weist die oben angegebene Struktur auf.
Ausbeute: 83,9 g (81,8 %)
Epoxidgehalt: 1,25 Aequ./kg (65,2 % der Theorie)
GPC: Mn = 1040, Mw = 1940

### Beispiel 13:

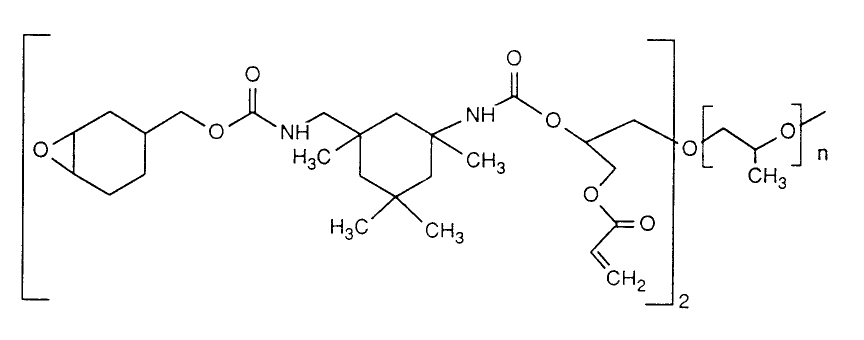

100 g (0,06 mol) des Produkts gemäss Beispiel 11 werden in 200 ml Chloroform gelöst, und auf 35°C erhitzt. 10 g Natriumacetat und 0,43 g Hydrochinonmonomethylether werden zugefügt. Dann werden 31,9 g (0,168 mol) 40 % Peressigsäure langsam zugetropft. Die Lösung wird noch 5 Stunden bei 35°C gerührt und wie in Beispiel 5 aufgearbeitet. Das erhaltene Produkt weist die oben angegebene Struktur auf.
Ausbeute: 84,4 g (82,8 %)
Epoxidgehalt: 1,05 Aequ./kg (89 % der Theorie)
GPC: Mn = 930, Mw = 1980

### Beispiel 14:

Folgende Komponenten werden bei 60°C in einem Rundkolben zu einer homogenen Zusammensetzung vermischt:
- 50 %: Produkt aus Beispiel 6
- 20 %: CY 179 (cycloaliphatisches Epoxidharz, Ciba-Geigy)
- 20 %: Sartomer 454 (ethoxyliertes Trimethylolpropantriacrylat)
- 8.4 %: Sartomer 213 (Butandioldiacrylat)
- 0.6 %: Cyracure UVI 6974
- 1 %: Irgacure 184

Die Zusammensetzung hat eine Viskosität von 443 cps bei 30°C. Mit Hilfe eines He-Cd-Lasers wird mit Hilfe des Baustils "Weave" ein Formkörper hergestellt, der einen Curl-Faktor von 0,5 % aufweist.

In der Technik der Stereolithographie wird als verfahrensspezifisches Mass für schwundinduzierte Deformationen der sogenannte "curl factor" angegeben (zur Messung des Curl-Faktors vgl. Proceedings 2^{nd} Int. Conference in Rapid Prototyping, Dayton, Ohio, (1991), oder P.F. Jacobs, Rapid Prototyping and Manufacturing, Soc. Manufact. Eng., 1992, p.40 ff.).
Der curl factor wird an stereolithographisch hergestellten Prüfkörpern ermittelt, wobei die Deformation eines freitragenden Teils des Prüf-Körpers durch Schrumpfung ermittelt wird. Der Curl-Faktor ist die Differenz der Höhen eines fixierten Teilstückes eines Probekörpers und eines nichtfixierten deformierten Teilstückes, dividiert durch die Länge des freitragenden Stückes, in %.

### Beispiel 15:

Folgende Komponenten werden bei 60°C in einem Rundkolben gemischt, bis eine homogene Zusammensetzung hergestellt ist.
- 33.5 %: Produkt aus Beispiel 5
- 15 %: SR 344 (Polyethylenglykol-400-diacrylat)
- 3 %: Pleximon V773 (Neopentylglykol-dimethacrylat)
- 2 %: SR 306 (Tripropylenglykol-diacrylat)
- 40 %: SR 348 (ethoxyliertes Bisphenol-A-dimethacrylat)
- 0.15 %: Hydrochinonmonomethylether
- 5.85 %: Irgacure 184
- 0.5 %: Cyracure UVI 6974

Die Zusammensetzung hat eine Viskosität von 1720 cps bei 30°C. Mit Hilfe eines He-Cd-Lasers werden Formkörper hergestellt, die nach der Laserhärtung einen E-Modul von 52 N/mm² aufweisen. Nach vollständiger Härtung (30 min UV, 30 min 130°C) wird ein E-Modul von 1640 N/mm² und eine Reissdehung von 2 % erhalten. Der Curl-Faktor (Baustil Weave) beträgt 15 %.

### Beispiel 16:

Folgende Komponenten werden bei 60°C in einem Rundkolben gemischt, bis eine homogene Zusammensetzung erreicht ist.
- 43 %: CY 179
- 30 %: Produkt gemäss Beispiel 7
- 25 %: Rapicure DVE 3 (Triethylenglykoldivinylether, GAF)
- 1 %: Irgacure 184
- 1 %: Cyracure UVI 6974

Die Zusammensetzung hat eine Viskosität von 274 cps bei 30°C. Mit Hilfe eines He-Cd-Lasers werden Formkörper hergestellt. Nach der Laserhärtung beträgt der E-Modul 3,1 N/mm², nach vollständiger Aushärtung (30 min UV, 30 min 130°C) werden ein E-Modul von 2240 N/mm² und eine Reissdehnung von 2,4 % gemessen.

### Beispiel 17:

Folgende Komponenten werden bei 60°C in einem Rundkolben gemischt bis eine homogene Zusammensetzung erreicht ist.
- 46 %: CY 179
- 10 %: DY 026 (Butandioldiglycidylether)
- 30 %: Produkt gemäss Beispiel 7
- 12 %: SR 399 (Dipentaerythritol-monohydroxy-pentaacrylat)
- 1 %: Irgacure 184
- 1 %: Cyracure UVI 6974

Die Zusammensetzung hat eine Viskosität von 2610 cps bei 30°C. Mit Hilfe eines He-Cd-Lasers werden Formkörper hergestellt. Nach der Laserhärtung beträgt der E-Modul 8,8 N/mm². Nach vollständiger Aushärtung (30 min UV, 30 min 130°C) werden ein E-Modul von 3021 N/mm² und eine Reissdehnung von 11 % gemessen.

### Beispiel 18:

Folgende Komponenten werden bei 60°C in einem Rundkolben gemischt, bis eine homogene Zusammensetzung erreicht ist.
- 33,5 %: Produkt gemäss Beispiel 8
- 15 %: SR 344
- 3 %: Pleximon V 773
- 2 %: SR 306
- 40 %: SR 348
- 0,15 %: Hydrochinonmonomethylether
- 5,85 %: Irgacure 184
- 0,5 %: Cyracure UVI 6974

Die Zusammensetzung hat eine Viskosität von 1610 cps bei 30°C. Mit Hilfe eines He-Cd-Lasers werden Formkörper hergestellt. Nach der Laserhärtung beträgt der E-Modul 50 N/mm². Nach vollständiger Aushärtung werden ein E-Modul von 2409 N/mm² und eine Reissdehnung von 4,2 % erhalten. Der Curl-Faktor (Baustil Weave) beträgt 15 %.

### Beispiel 19:

Folgende Komponenten werden bei 60°C in einem Rundkolben gemischt, bis eine homogene Zusammensetzung erreicht ist.
- 33,5 %: Produkt gemäss Beispiel 9
- 15 %: SR 344
- 3 %: Pleximon V 773
- 2%: SR 306
- 40 %: SR 348
- 0,15 %: Hydrochinonmonomethylether
- 5,85 %: Irgacure 184
- 0,5 %: Cyracure UVI 6974.

Die Zusammensetzung hat eine Viskosität von 4310 cps bei 30°C. Mit Hilfe eines He-Cd-Lasers werden Formkörper hergestellt. Nach der Laserhärtung beträgt der E-Modul 40 N/mm². Nach vollständiger Aushärtung wird ein E-Modul von 2126 N/mm² und eine Reissdehnung von 2 % erhalten. Der Curl-Faktor (Baustil Weave) beträgt 14 %.

### Beispiel 20:

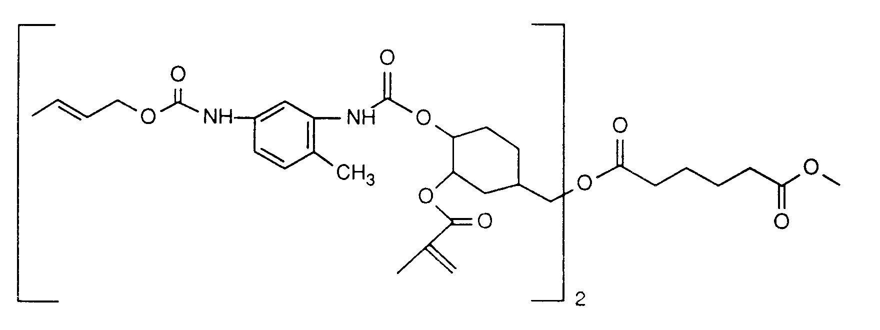

64,92 g (0,372 mol) Toluylendiisocyanat, 0,47 g Dibutylzinndilaurat, 0,5 g Ralox® 46 und 100 ml Toluol werden in einem Reaktionsgefäss unter Rühren auf 35°C erwärmt. 100 g (0,186 mol) des Reaktionsprodukts aus Araldit CY 177 und Methacrylsäure (siehe Beispiel 2) werden langsam zugetropft. Die exotherme Reaktion wird mit einem Eisbad auf 35°C gehalten. Nach ca. 10 Stunden wird ein Isocyanatgehalt von 1,54 Aequ./kg erhalten. Dann werden 26,83 g (0,372 mol) Crotylalkohol zugetropft und so lange gerührt, bis ein Isocyanatgehalt von 0,06 Aequ./kg erreicht wird (ca. 64 Stunden). Das Lösungsmittel wird im Hochvakuum abdestilliert. Das erhaltene Produkt weist die oben angegebene Struktur auf.
GPC: Mn = 1390, Mw = 2990

### Beispiel 21:

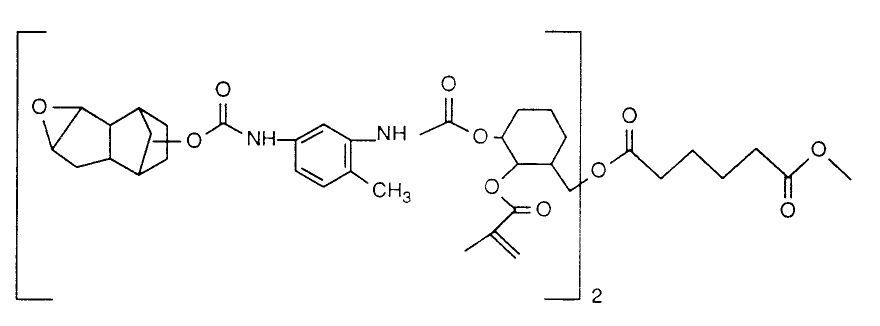

64,8 g (0,372 mol) Toluylendiisocyanat, 0,47 g Dibutylzinndilaurat, 0,5 g Ralox® 46 und 100 ml Toluol werden in einem Reaktionsgefäss unter Rühren auf 35°C erwärmt. 100 g (0,186 mol) des Reaktionsprodukts aus Araldit CY 177 und Methacrylsäure (siehe Beispiel 2), verdünnt mit 50 ml Toluol, werden langsam zugetropft, wobei die Temperatur bei 35°C gehalten wird. Nach ca. 3 Stunden wird ein Isocyanatgehalt von 1,47 Aequ./kg erhalten. Dann werden 61,83 g (0,372 mol) epoxidierter TCD-Alkohol (hergestellt durch Oxidation von TCD-Alkohol E mit 40 % Peressigsäure) hinzugetropft und so lange gerührt, bis ein Isocyanatgehalt von 0,019 Aequ./kg erhalten wird (ca. 26 Stunden). Das Lösungsmittel wird im Hochvakuum abdestilliert. Das erhaltene Produkt weist die oben angegebene Struktur auf.
GPC: Mn = 1150, Mw = 2530

## Patentansprüche

1. Urethangruppenhaltige (Meth)Acrylate der Formel I 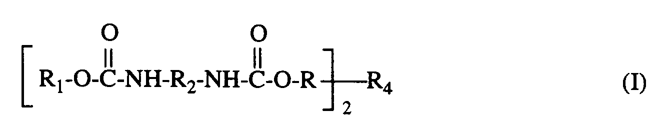 worin
R eine zweiwertige Gruppe der Formeln 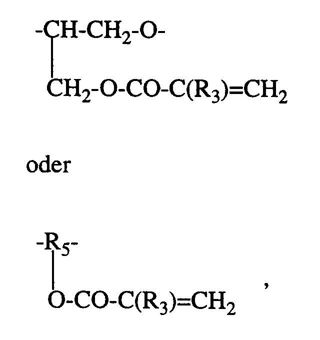
R₁ eine kationisch polymerisierbare Gruppe der Formeln 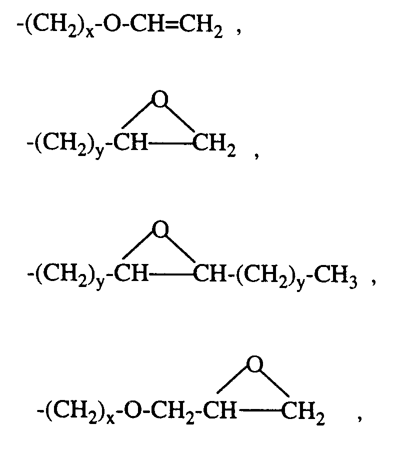 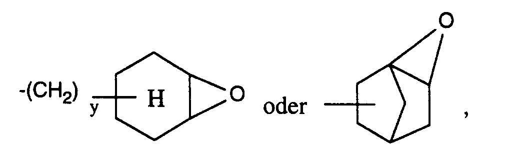 oder eine radikalisch polymerisierbare Gruppe der Formeln 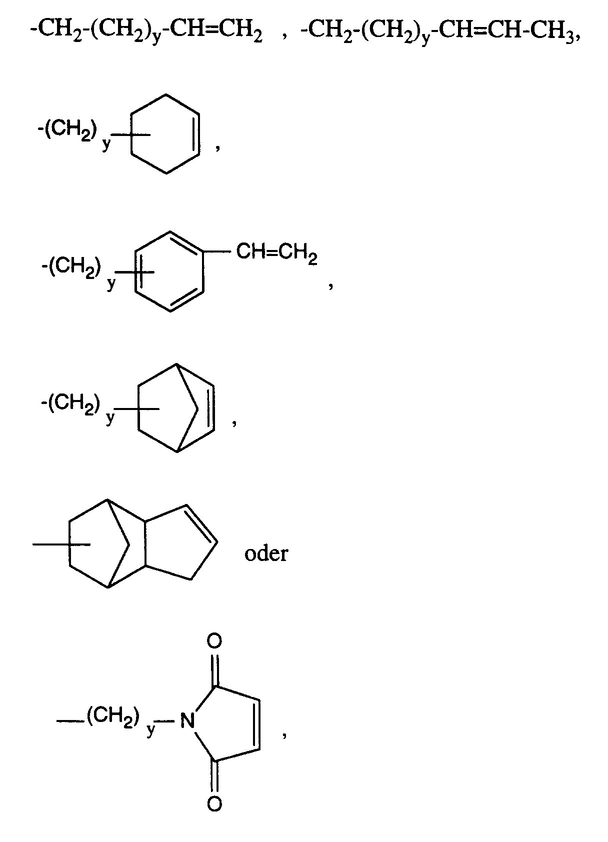 worin x eine ganze Zahl von 2 bis 20 und y eine ganze Zahl von 1 bis 20 bedeuten,
R₂ einen aliphatischen, cycloaliphatischen oder aromatischen Rest,
R₃ Wasserstoff oder CH₃,
R₄ den Rest einer aliphatischen, cycloaliphatischen oder aromatischen Diglycidylverbindung nach Entfernen des Diglycidylrestes, oder den Rest eines cycloaliphatischen Diepoxides und
R₅ ein cycloaliphatisches Brückenglied bedeuten.

2. Urethangruppenhaltige (Meth)Acrylate gemäss Anspruch 1, worin R₁ eine kationisch polymerisierbare Gruppe bedeutet.

3. Urethangruppenhaltige (Meth)Acrylate der Formel I gemäss Anspruch 1, worin R₂ und R₄ unabhängig voneinander ein C₄-C₂₀ aliphatisches Brückenglied, das durch mindestens eine Ether-, Ester-, Urethan-, Amid- oder Arylengruppe unterbrochen sein kann, und ferner ein cycloaliphatisches oder ein aromatisches Brückenglied bedeuten.

4. Urethangruppenhaltige (Meth)Acrylate gemäss Anspruch 3, worin R₂ ein C₄-C₂₀-aliphatisches oder ein aromatisches Brückenglied oder mehrere aromatische Gruppen, die durch aliphatische Gruppen unterbrochen sind, bedeutet.

5. Urethangruppentaltige (Meth)Acrylate gemäss Anspruch 3, worin R₄ ein C₄-C₂₀ aliphatisches Brückenglied, das durch mindestens eine Ether- oder eine Estergruppe unterbrochen ist, oder ein aromatisches Brückenglied, bedeutet.

6. Urethangruppenhaltige (Meth)Acrylate gemäss Anspruch 1, worin x und y eine ganze Zahl von 1 bis 6 bedeuten.

7. Verfahren zur Herstellung der urethangruppenhaltigen (Meth)Acrylate der Formel I gemäss Anspruch 1, dadurch gekennzeichnet, dass ein Epoxyacrylat der Formel II
[ HO-R⁆₂R₄ (II)
mit einem Diisocyanat der Formel III
OCN-R₂-NCO (III)
in äquimolarer Menge in einem organischen Lösungsmittel in Gegenwart eines Katalysators und eines Inhibitors zur Reaktion gebracht werden und die entstandene Verbindung der Formel IV
[ OCN-R₂-NH-CO-O-R⁆₂R₄ (IV)
mit einer den Rest R₁ einführenden Verbindung umgesetzt wird, wobei R₁, R₂ und R₄ die im Anspruch 1 angegebene Bedeutung haben.

8. Verfahren gemäss Anspruch 7, dadurch gekennzeichnet, dass die erhaltenen urethangruppenhaltigen (Meth)Acrylate oxydiert werden.

9. Zusammensetzung enthaltend
a) 5 bis 99 Gew.% eines monomeren polymerisierbaren urethangruppenhaltigen (Meth)Acrylates der Formel I,
b) 1 bis 10 Gew.% eines radikalischen Photoinitiators,
c) 0 bis 20 Gew.% üblicher Additive,
d) 0 bis 80 Gew.% eines oder mehrerer mono-, di- oder mehrfunktionellen (Meth)Acrylate oder Gemische davon,
e) 0 bis 80 Gew.% eines di- oder mehrfunktionellen Epoxides oder Gemische davon,
f) 0 bis 50 Gew.% eines OH-terminierten Polyethers oder Polyesters, oder Gemische davon,
g) 0 bis 5 Gew.% eines kationischen Photoinitiators, und
h) 0 bis 80 Gew.% eines oder mehrerer mono-, di- oder mehrfunktionellen Vinylether,
wobei der Anteil der Komponenten a) bis h) zusammen 100 Gew.% beträgt.

10. Verwendung der Zusammensetzungen gemäss Anspruch 9 in der Stereolithographie.

11. Verfahren zum Polymerisieren der Zusammensetzungen gemäss Anspruch 9, indem man diese mit aktinischem Licht bestrahlt.

12. Verfahren zur Herstellung von dreidimensionalen Gegenständen aus einer Zusammensetzung gemäss Anspruch 9 mittels einem lithographischen Verfahren, wobei die Oberfläche einer Schicht dieser Zusammensetzung ganzflächig oder in einem vorbestimmten Muster mit einer UV- und/oder VIS-Lichtquelle bestrahlt wird, so dass sich in den bestrahlten Bereichen eine Schicht in einer gewünschten Schichtdicke verfestigt, dann eine neue Schicht aus dieser Zusammensetzung auf der verfestigten Schicht gebildet wird, die ebenfalls ganzflächig oder in einem vorbestimmten Muster bestrahlt wird, und wobei durch wiederholtes Beschichten und Bestrahlen dreidimensionale Gegenstände aus mehreren aneinander haftenden, verfestigten Schichten erhalten werden.

13. Verfahren gemäss Anspruch 12, wonach als Lichtquelle ein Laserstrahl, vorzugsweise ein computergesteuerter Laserstrahl, verwendet wird.

## Claims

1. A (meth)acrylate of the formula I containing urethane groups 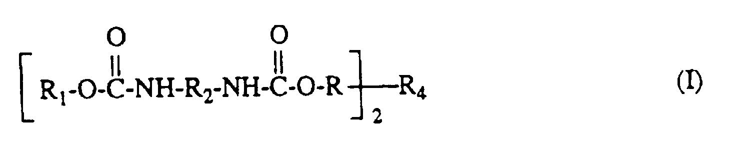 in which
R is a divalent group of the formula 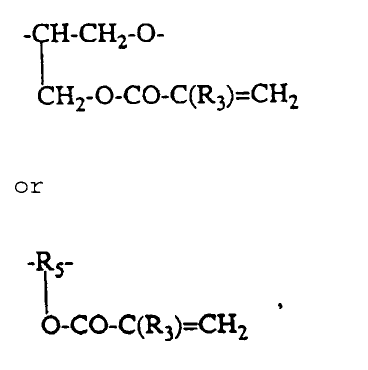
R₁ is a cationically polymerizable group of the formula 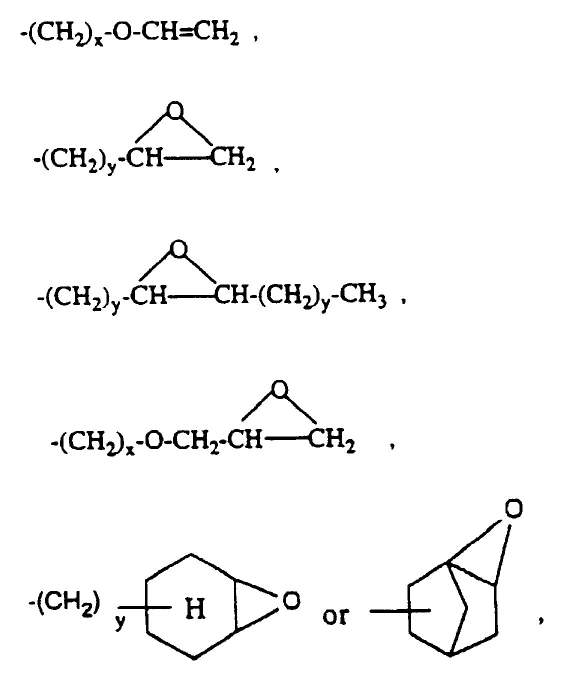 or a free-radical-polymerizable group of the formula 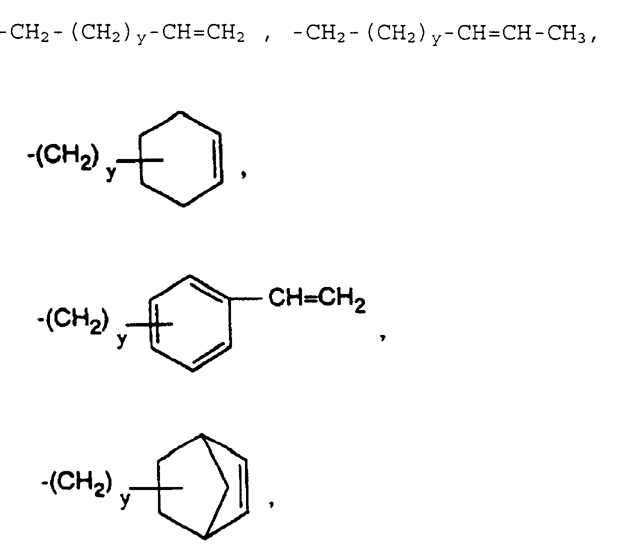 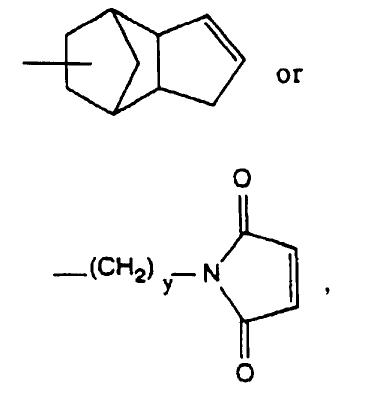 in which x is an integer from 2 to 20, and y is an integer from 1 to 20,
R₂ is an aliphatic, cycloaliphatic or aromatic radical,
R₃ is hydrogen or CH₃,
R₄ is the radical of an aliphatic, cycloaliphatic or aromatic diglycidyl compound after removal of the diglycidyl radical, or is the radical of a cycloaliphatic diepoxide, and
R₅ is a cycloaliphatic bridge.

2. A (meth)acrylate containing urethane groups according to claim 1, in which R₁ is a cationically polymerizable group.

3. A (meth)acrylate of the formula I containing urethane groups according to claim 1, in which R₂ and R₄, independently of one another, are a C₄-C₂₀aliphatic bridge, which may be interrupted by at least one ether, ester, urethane, amide or arylene group, and furthermore are a cycloaliphatic or an aromatic bridge.

4. A (meth)acrylate containing urethane groups according to claim 3, in which R₂ is a C₄-C₂₀aliphatic or an aromatic bridge or a plurality of aromatic groups interrupted by aliphatic groups.

5. A (meth)acrylate containing urethane groups according to claim 3, in which R₄ is a C₄-C₂₀aliphatic bridge interrupted by at least one ether or ester group, or is an aromatic bridge.

6. A (meth)acrylate containing urethane groups according to claim 1, in which x and y are integers from 1 to 6.

7. A process for the preparation of a (meth)acrylate of the formula I containing urethane groups according to claim 1, which comprises reacting an epoxyacrylate of the formula II
[ HO-R⁆₂R₄ (II)
with a diisocyanate of the formula III
OCN-R₂-NCO (III)
in equimolar amount in an organic solvent in the presence of a catalyst and in the presence of an inhibitor, and reacting the resultant compound of the formula IV
[ OCN-R₂-NH-CO-O-R⁆₂R₄ (IV)
with a compound which introduces the radical R₁, where R₁, R₂ and R₄ are as defined in claim 1.

8. A process according to claim 7, wherein the resultant (meth)acrylate containing urethane groups is oxidized.

9. A composition comprising
a) from 5 to 99% by weight of a monomeric, polymerizable (meth)acrylate of the formula I containing urethane groups,
b) from 1 to 10% by weight of a free-radical photoinitiator,
c) from 0 to 20% by weight of conventional additives,
d) from 0 to 80% by weight of one or more mono-, di- or polyfunctional (meth)acrylates or mixtures thereof,
e) from 0 to 80% by weight of a di- or polyfunctional epoxide, or mixtures thereof,
f) from 0 to 50% by weight of an OH-terminated polyether or polyester, or mixtures thereof,
g) from 0 to 5% by weight of a cationic photoinitiator, and
h) from 0 to 80% by weight of one or more mono-, di- or polyfunctional vinyl ethers, where the proportion of components a) to h) together is 100% by weight.

10. The use of a composition according to claim 9 in stereolithography.

11. A process for the polymerization of a composition according to claim 9, in which the latter is irradiated with actinic light.

12. A process for the production of three-dimensional objects from a composition according to claim 9 by a lithographic process, where the surface of a layer of this composition is irradiated over the entire area or in a premeditated pattern with a UV and/or VIS light source so that a layer solidifies in the desired layer thickness in the irradiated areas, a new layer of this composition is then formed on the solidified layer and is likewise irradiated over the entire area or in a predetermined pattern, and where three-dimensional objects comprising a plurality of mutually adherent, solidified layers are obtained by repeated coating and irradiation.

13. A process according to claim 12, in which the light source used is a laser beam, preferably a computer-controlled laser beam.

## Revendications

1. (Méth)acrylates à groupes uréthanne de formule I 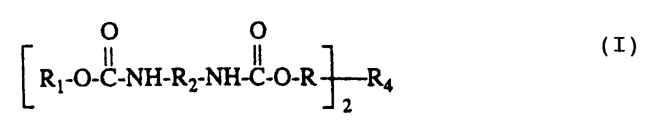 où
R représente un groupe bifonctionnel de formules 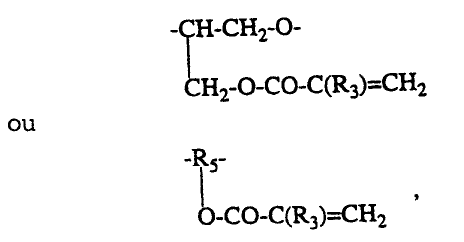
R₁ représente un groupe polymérisable par voie cationique de formules 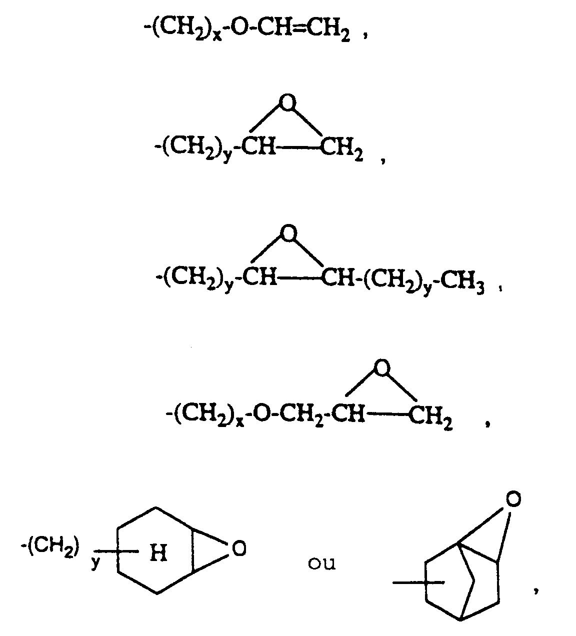 ou un groupe polymérisable par voie radicalaire de formules 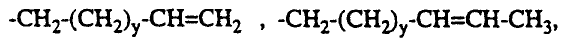 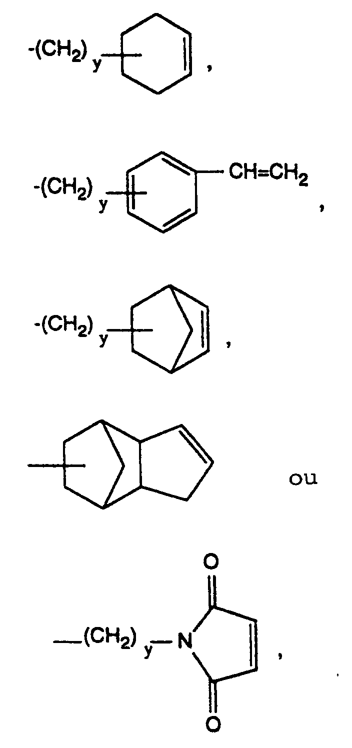 où
x est un entier de 2 à 20 et
y est un entier de 1 à 20,
R₂ représente un reste aliphatique, cycloaliphatique ou aromatique,
R₃ représente un atome d'hydrogène ou CH₃,
R₄ représente le reste d'un composé diglycidylique aliphatique, cycloaliphatique ou aromatique après avoir éliminé le reste glycidyle, ou le reste d'un diépoxyde cycloaliphatique et
R₅ représente un élément de pontage cycloaliphatique.

2. (Méth)acrylates à groupes uréthanne selon la revendication 1, où R₁ est un groupe polymérisable par voie cationique.

3. (Méth)acrylates à groupes uréthanne de formule I selon la revendication 1, où R₂ et R₄ représentent chacun, indépendamment l'un de l'autre, un élément de pontage aliphatique en C₄-C₂₀ qui peut être interrompu par au moins un groupe éther, ester, uréthanne, amide ou arylène, et de plus un élément de pontage cycloaliphatique ou aromatique.

4. (Méth)acrylates à groupes uréthanne selon la revendication 3, où R₂ représente un élément de pontage aliphatique en C₄-C₂₀ ou aromatique ou plusieurs groupes aromatiques qui sont interrompus par des groupes aliphatiques.

5. (Méth)acrylates à groupes uréthanne selon la revendication 3, où R₄ représente un élément de pontage aliphatique en C₄-C₂₀ qui peut être interrompu par au moins un groupe éther ou ester ou représente un élément de pontage aromatique.

6. (Méth)acrylates à groupes uréthanne selon la revendication 1, où x et y sont un entier de 1 à 6.

7. Procédé de préparation de (méth)acrylates à groupes urétahnne de formule I selon la revendication 1, caractérisé en ce qu'on fait agir un époxyacrylate de formule II
[ HO-R⁆₂R₄ (II)
sur un diisocyanate de formule III
OCN-R₂-NCO (III)
de façon appropriée en quantités équimolaires dans un solvant organique en présence d'un catalyseur et d'un inhibiteur, puis en faisant réagir le composé obtenu de formule IV
[ OCN-R₂-NH-CO-O-R⁆₂R₄ (IV)
sur un composé introduisant le reste R₁, R₁, R₂ et R₄ possédant les significations données à la revendication 1.

8. Procédé selon la revendication 7, caractérisé en ce que les (méth)acrylates à groupes uréthanne obtenus sont oxydés.

9. Composition contenant
a) de 5 à 99% massiques d'un (méth)acrylate à groupes uréthanne, monomère, polymérisable, de formule I,
b) de 1 à 10% massiques d'un photoamorceur radicalaire,
c) de 0 à 20% massiques d'additifs usuels,
d) de 0 à 80% massiques d'un ou plusieurs (méth)acrylates mono-, di- ou multifonctionnels ou leurs mélanges,
e) de 0 à 80% massiques d'un ou plusieurs époxydes di- ou multifonctionnels,
f) de 0 à 50% massiques d'un polyéther ou polyester à groupes terminal OH ou leurs mélanges,
g) de 0 à 5% massiques d'un photoamorceur cationique, et
h) de 0 à 80% massiques d'un ou plusieurs éthers vinyliques mono-, di- ou multifonctionnels, le taux en constituants a) à h) ensemble étant égal à 100% massiques.

10. Utilisation des compositions selon la revendication 9 en stéréolithographie.

11. Procédé de polymérisation des compositions selon la revendication 9 en les irradiant par une lumière actinique.

12. Procédé de préparation d'objets tridimensionnels à partir d'une composition selon la revendication 9 à l'aide d'un procédé lithographique, où la surface d'une couche de cette composition est irradiée sur la totalité ou selon in modèle prédéterminé par une source de lumière UV et/ou VIS de façon telle qu'une couche d'une épaisseur voulue se consolide sur les domaines irradiés, puis on forme sur la couche consolidée une nouvelle couche à partir de cette composition, laquelle est également irradiée sur la totalité de la surface ou selon un modèle prédéterminé et par revêtement et irradiation répétés, on obtient des objets tridimensionnels qui sont un assemblage de plusieurs couches consolidées, adhérentes l'une à l'autre.

13. Procédé selon la revendication 12, selon lequel on utilise comme source de lumière un rayon laser, de préférence un rayon laser piloté par ordinateur.
